(19) Europäisches Patentamt
European Patent Office
Office européen des brevets

(11) Veröffentlichungsnummer: **0 251 262**
**A2**

(12) # EUROPÄISCHE PATENTANMELDUNG

(21) Anmeldenummer: **87109289.6**

(51) Int. Cl.4: **C07C 33/42** , C07C 29/58

(22) Anmeldetag: **27.06.87**

(30) Priorität: **04.07.86 DE 3622533**

(43) Veröffentlichungstag der Anmeldung: **07.01.88 Patentblatt 88/01**

(84) Benannte Vertragsstaaten: **BE CH DE FR GB IT LI NL**

(71) Anmelder: **HOECHST AKTIENGESELLSCHAFT**
**Postfach 80 03 20**
**D-6230 Frankfurt am Main 80(DE)**

(72) Erfinder: **Raab, Klaus, Dr.**
**Schneibsteinstrasse 6**
**D-8269 Burgkirchen(DE)**
Erfinder: **Pöschl, Johanna**
**Dorfstrasse 2**
**D-8261 Halsbach(DE)**

(54) **Verfahren zur Herstellung von Perfluoralkylalkenolen.**

(57) Beim neuen Verfahren zur Herstellung von Perfluoralkylalkenolen werden Perfluoralkylhalogenhydrine mit speziellen ionischen Basen, nämlich mit Alkalimetallcarbonat, Alkalimetallhydrogencarbonat oder Alkylammoniumhydrogencarbonat, in Gegenwart aprotischer und polarer Lösungsmittel dehydrohalogeniert. Mit diesem Verfahren werden hohe Ausbeuten an den angestrebten Perfluoralkylalkenolen erreicht und es werden, wenn überhaupt, nur sehr wenig unerwünschte Nebenprodukte wie teilfluorierte Etheralkohole und/oder teilfluorierte Epoxide gebildet.

EP 0 251 262 A2

Xerox Copy Centre

## Verfahren zur Herstellung von Perfluoralkylalkenolen

Die Erfindung betrifft. ein Verfahren zur Herstellung von Perfluoralkylalkenolen der nachstehenden Formel I

$R_F-CH=CH-(CH_2)_n-OH$ ,

worin $R_F$ ein Perfluoralkylrest mit 1 bis 18 C-Atomen und n eine ganze Zahl von 1 bis 6 ist, durch Umsetzung eines Perfluoralkylhalogenhydrins der nachstehenden Formel II

$R_F-CH_2-CH(Hal)-(CH_2)_n-OH$ ,

worin $R_F$ und n die obengenannte Bedeutung haben und Hal für ein Halogen steht, mit ionischen Basen in Gegenwart eines Lösungsmittels.

Ein solches Verfahren ist aus der US-Patentschrift 3 285 975 und aus der britischen Patentschrift 1 101 049 bekannt. Als Basen für die Umsetzung (Dehydrohalogenierung) des Perfluoralkylhalogenhydrins werden KOH, NaOH oder Alkalimetallalkoholate wie Natriummethylat und Kaliumethylat eingesetzt und als Lösungsmittel Methanol, Ethanol oder Gemische dieser Alkohole mit Wasser. Dieses Verfahren weist den Nachteil auf, daß es die angestrebten Perfluoralkylalkenole in einer relativ geringen Ausbeute liefert. Darüber hinaus verläuft die Umsetzung unter Bildung einer mehr oder weniger großen Menge von unerwünschten Nebenprodukten wie teilfluorierten Etheralkoholen der Formel $R_F-CH_2-CH(OH)-CH_2-OCH_3$ und/oder teilfluorierten Epoxiden der Formel

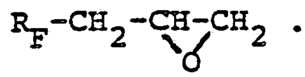

Aus der japanischen Kokai JP 52-144610 (Derwent-Referat 05434 A) ist ein Verfahren zur Herstellung von Perfluoralkylpropenol bekannt (diese Verbindung ergibt sich, wenn in der genannten Formel I n = 1 ist), bei dem ein Perfluoralkyliodpropanol (diese Verbindung ergibt sich, wenn in der genannten Formel II Hal = I ist) mit einem speziellen tertiären Amin umgesetzt wird, in Gegenwart eines Lösungsmittels mit einer niedrigen Dielektrizitätskonstanten, wie Diethylether, Dimethylglykol, Dioxan, Tetrahydrofuran, Methylacetat und/oder Methylethylketon (das sind aprotische Lösungsmittel) oder in Gegenwart eines Lösungsmittels mit einer hohen Dielektrizitätskonstanten wie Methanol, Ethanol, Dimethylformamid und/oder Dimethylsulfoxid. Der besondere Nachteil dieses Verfahrens liegt darin, daß es auf einem speziellen tertiären Amin als der zweiten Reaktionskomponente beruht. Dazu kommt, daß auch bei diesem Verfahren die oben erwähnten, unerwünschten Nebenprodukte, insbesondere die teilfluorierten Epoxide, nämlich Perfluoralkyl-1,2-epoxypropane, gebildet werden; diese Bildung tritt vor allem dann auf, wenn die Lösungsmittel mit der hohen Dielektrizitätskonstanten eingesetzt werden.

Der Erfindung liegt demnach die Aufgabe zugrunde, das eingangs genannte Verfahren zur Herstellung von Perfluoralkylalkenolen, bei dem als zweite Reaktionskomponente einfache basische Verbindungen (ionische Basen) eingesetzt werden, dahingehend zu verbessern, daß die angestrebten Alkenole in einer hohen Ausbeute erhalten werden und bei der Umsetzung keine nennenswerten Mengen an den genannten oder anderen Nebenprodukten entstehen.

Das erfindungsgemäße Verfahren zur Herstellung von Perfluoralkylalkenolen der nachstehenden Formel I

$R_F-CH=CH-(CH_2)_n-OH$ ,

worin $R_F$ ein Perfluoralkylrest mit 1 bis 18 C-Atomen und n eine ganze Zahl von 1 bis 6 ist, durch Umsetzung (Dehydrohalogenierung) eines Perfluoralkylhalogenhydrins der nachstehenden Formel II

$R_F-CH_2-CH(Hal)-(CH_2)_n-OH$ ,

worin $R_F$ und n die genannte Bedeutung haben und Hal für ein Halogen steht, mit ionischen Basen in Gegenwart eines Lösungsmittels, ist dadurch gekennzeichnet, daß das Perfluoralkylhalogenhydrin mit einem

Alkalimetallcarbonat im Molverhältnis von 1 : 0,5 bis 1,5 oder mit einem Alkalimetallhydrogencarbonat oder einem Alkylammoniumhydrogencarbonat im Molverhältnis von 1 : 1,01 bis 2 in Gegenwart eines aprotischen und polaren Lösungsmittels umgesetzt wird, bis im wesentlichen kein Kohlendioxid mehr entsteht, und aus dem Umsetzungsprodukt die angestrebte Perfluoralkylalkenol-Verbindung isoliert wird, mit der Maßgabe, daß bei Einsatz von Alkalimetallcarbonat und Alkalimetallhydrogencarbonat die Umsetzung bei einer Temperatur von 80 bis 150 °C und bei Einsatz von Alkylammoniumhydrogencarbonat die Umsetzung bei einer Temperatur von -5 bis 90 °C durchgeführt wird.

Das Perfluoralkylhalogenhydrin und das Alkalimetallcarbonat werden erfindungsgemäß im Molverhältnis von 1 : 0,5 bis 1,5, vorzugsweise 1 : 0,6 bis 1, eingesetzt. Das Perfluoralkylhalogenhydrin und das Alkalimetallhydrogencarbonat sowie das Alkylammoniumhydrogencarbonat werden im Molverhältnis von 1 : 1,01 bis 2, vorzugsweise 1 : 1,1 bis 1,5, eingesetzt.
Bei Einsatz von Alkalimetallcarbonat und bei Einsatz von Alkalimetallhydrogencarbonat wird die erfindungsgemäße Umsetzung, das ist die Dehydrohalogenierung des Perfluoralkylhalogenhydrins, bei einer Temperatur von 80 bis 150 °C, vorzugsweise 90 bis 130 °C, vorgenommen. Bei Einsatz von Alkylammoniumhydrogencarbonat als dem Dehydrohalogenie rungsmittel wird die Umsetzung bei einer Temperatur von -5 bis 90 °C vorgenommen. Aufgrund der eingesetzten Carbonate und Hydrogencarbonate entsteht bei der erfindungsgemäßen Umsetzung Kohlendioxid. Das Ende der Umsetzung wird durch die Abnahme und das Aufhören der Kohlendioxid-Bildung angezeigt. Die Reaktionszeit der erfindungsgemäßen Umsetzung liegt im allgemeinen bei 5 bis 20 h.

Wie sich herausgestellt hat, verläuft die Umsetzung von Perfluoralkylhalogenhydrin und Alkylammoniumhydrogencarbonat dann besonders vorteilhaft im Hinblick auf eine hohe Ausbeute an angestrebter Verbindung, wenn eine auf das eingesetzte Perfluoralkylhalogenhydrin speziell abgestimmte Umsetzungstemperatur eingehalten wird. So ist es besonders vorteilhaft, wenn im Falle eines Perfluoralkylhalogenhydrins der obengenannten Formel II mit n = 1 (das ist ein 3-Perfluoralkyl-2-halogenpropanol-1) zunächst eine Temperatur von -5 bis 25 °C, vorzugsweise 0 bis 20 °C, eingehalten wird, bis das Perfluoralkylhalogenpropanol im wesentlichen verbraucht ist und anschließend eine Temperatur von 40 bis 90 °C, vorzugsweise 50 bis 70 °C, bis im wesentlichen kein Kohlendioxid mehr entsteht. Bei dieser, hinsichtlich Temperatur zweistufigen Reaktionsführung entsteht in der ersten Stufe ein cyclisches Carbonat als Zwischenprodukt, das in der zweiten Stufe, das heißt bei höherer Reaktionstemperatur, in Gegenwart von noch anwesendem Alkylammoniumhydrogencarbonat (es genügt eine katalytische Menge) in das angestrebte Perfluoralkylpropenol übergeführt wird; die Ausbeute an Perfluoralkylpropenol ist besonders groß. Wird die Umsetzung von Anfang an bei der höheren Temperatur vorgenommen, also nicht über das Zwischenprodukt geführt, wird neben dem Perfluoralkylpropenol auch unerwünschtes Epoxid gebildet. Die nachstehenden Reaktions gleichungen sollen die zweistufige Umsetzung veranschaulichen ($R_F$ und Hal haben die obengenannte Bedeutung):

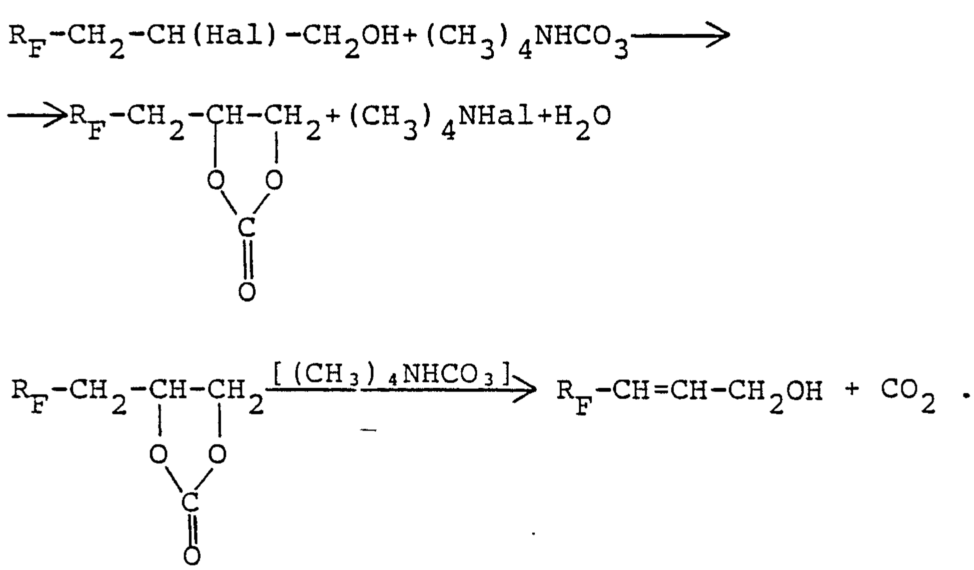

Die bei der angegebenen niedrigen Temperatur erhaltenen polyfluorierten , cyclischen Carbonate werden im Rahmen der vorliegenden Erfindung nicht isoliert, sondern durch das Erhitzen auf die angegebene höhere Temperatur und das im Reaktionsgemisch noch vorhandene Alkylammoniumhydrogencarbonat (es genügen katalytische Mengen) in das angestrebte 3-Perfluoralkyl-2-propen-1-ol übergeführt.

Im Falle der Umsetzung von Alkylammoniumhydrogencarbonat und einem Perfluoralkylhalogenhydrin der eingangs genannten Formel mit n = 2 bis 6 ist es vorteilhaft, eine Umsetzungstemperatur von 40 bis 90 °C, vorzugsweise 50 bis 70 °C, einzuhalten. Das Führen der Umsetzung über Zwischenprodukte ist hier ebenso wie bei den Umsetzungen von Perfluoralkylhalogenhydrin mit Alkalimetallcarbonat und Alkalimetallhydrogencarbonat zur Erreichung einer sehr hohen Ausbeute an angestrebtem Perfluoralkylalkenol nicht erforderlich;die vergleichsweise niedrige Temperatur von 40 bis 90 °C, vorzugsweise 50 bis 70 °C, resultiert daraus, daß Alkylammoniumhydrogencarbonate reaktiver sind als Alkalimetallcarbonate und Alkalimetallhydrogencarbonate.

Der Perfluoralkylrest $R_F$ in den Formeln I und II ist ein unverzweigter oder verzweigter Alkylrest, vorzugsweise ein unverzweigter Alkylrest, mit 1 bis 18 C-Atomen, vorzugsweise 2 bis 10 C-Atomen. Beim Perfluoralkyl kann es sich auch um eine Mischung von Perfluoralkyl-Gruppen handeln. n ist eine ganze Zahl von 1 bis 6, vorzugsweise 1 bis 2, insbesondere 1. Hal steht in Formeln I und II für Halogen, vorzugsweise Brom oder Iod. Beispiele für $R_F$-Reste sind $CF_3$-, $CF_3$-$CF_2$-, $(CF_3)_2CF$-, $CF_3$-$(CF_2)_3$-, $CF_3$-$(CF_2)_n$-, worin n 5, 6, 7, 8, 9, 11, 13 oder 15 ist, $(CF_3)_2CF$-$(CF_2)_2$-, $(CF_3CF_2CF_2)$ $(CF_3)_2C$-, $(CF_3)_2CF$-$(CF_2)_4$- ; $HCF_2$-$CF_2$-, $HCF_2$-$(CF_2)_3$-.

Die erfindungsgemäß einzusetzenden Perfluoralkylhalogenhydrine sind schon seit langem bekannt. Bei den erfindungsgemäß einzusetzenden Alkalimetallcarbonaten und Alkalimetallhydrogencarbonaten handelt es sich vorzugsweise um die Natrium-und Kaliumverbindung. Der Alkylrest in den erfindungsgemäß einzusetzenden Alkylammoniumhydrogencarbonaten ist vorzugsweise eine $C_1$-bis $C_4$-Alkylgruppe und insbesondere Methyl oder Ethyl. Von den Alkylammoniumhydrogencarbonaten sind die Tetraalkylammoniumhydrogencarbonate bevorzugt.

Die erfindungsgemäße Umsetzung wird in Gegenwart eines aprotischen und polaren Lösungsmittels durchgeführt. Geeignete derartige Lösungsmittel sind Dimethylformamid, Dimethylacetamid, N-Methylpyrrolidon, Dimethylsulfoxid, Sulfolan, Tetramethylharnstoff und Acetonitril, wobei Dimethylformamid, Dimethylacetamid, N-Methylpyrrolidon und Acetonitril bevorzugt sind. Es hat sich herausgestellt, daß bei Einsatz von Alkalimetallcarbonat und Alkalimetall hydrogencarbonat Dimethylformamid, Dimethylacetamid und N-Methylpyrrolidon besonders geeignete Lösungsmittel sind, und bei Einsatz von Alkylammoniumhydrogencarbonat Acetonitril das besonders geeignete Lösungsmittel ist. Die Menge an Lösungsmittel, in dem die Perfluoralkylhalogenhydrine im wesentlichen löslich und die Carbonate und Hydrogencarbonate im wesentlichen unlöslich sind, kann in weiten Grenzen variieren. Aus Zweckmäßigkeitsgründen wird soviel Lösungsmittel genommen, daß aus eingesetztem Halogenhydrin, Carbonat oder Hydrogencarbonat und Lösungsmittel eine gut rührbare Suspension vorliegt. In der Regel werden 0,1 bis 15 l Lösungsmittel, vorzugsweise 0,2 bis 2 l, pro mol Perfluoralkylhalogenhydrin eingesetzt.

Die erfindungsgemäße Umsetzung wird in der Regel in der Weise durchgeführt, daß die Ausgangsverbindungen und das Lösungsmittel in ein Reaktionsgefäß eingebracht werden und die erhaltene Suspension unter Rühren auf Reaktionstemperatur gebracht wird, worauf die Umsetzung abläuft. Im Falle von Reaktionstemperaturen, die unterhalb Raumtemperatur liegen, wird eine entsprechende Abkühlung der Ausgangsverbindungen, des Lösungsmittels und der Suspension vorgenommen. Der Verlauf der Umsetzung kann an der obengenannten Kohlendioxid-Entwicklung und/oder NMR-spektroskopisch verfolgt werden.

Die Isolierung des angestrebten Perfluoralkylalkenols nach Beendigung der Umsetzung wird bevorzugt in der Weise durchgeführt, daß das Umsetzungsprodukt in Wasser aufgenommen wird, wobei eine organische Phase und eine wäßrige Phase gebildet wird. Die organische Phase besteht im wesentlichen aus dem angestrebten Perfluoralkylalkenol und die wäßrige Phase besteht im wesentlichen aus Wasser und den vorhandenen wasserlöslichen Verbindungen. Nach Abtrennen der organischen Phase kann diese destillativ behandelt werden, um ein besonders reines Perfluoralkylalkenol zu erhalten.

Mit dem erfindungsgemäßen Verfahren werden die angestrebten Perfluoralkylalkenole in einer hohen Ausbeute erhalten. Mit diesem Verfahren fallen auch keine oder keine nennenswerten Mengen an unerwünschten Nebenprodukten an. Bei den erfindungsgemäß einzusetzenden Basen, den Alkalimetallcarbonaten, Alkalimetallhydrogencarbonaten und Alkylammoniumhydrogencarbonaten, als der zweiten Reaktionskomponente neben dem Perfluoralkylhalogenhydrin, handelt es sich um ganz einfache Verbindungen, was einen weiteren großen Vorteil des erfindungsgemäßen Verfahrens darstellt.

Die Erfindung wird nun an Beispielen noch näher erläutert.

Beispiel 1

In einem 500-ml-Dreihalskolben mit Rückflußkühler, Rührer und Innenthermometer werden 60,8 g (0,2 mol) $CF_3CF_2$-$CH_2$-CHI-$CH_2OH$, 30 g (0,3 mol) $KHCO_3$ und 200 ml N-Methylpyrrolidon eingebracht. Die Suspension wird 14 h lang bei 100 °C gerührt,bis die $CO_2$-Entwicklung beendet ist. Nach Abkühlen auf Raumtemperatur wird der Kolbeninhalt filtriert. Das Filtrat wird mit ca. 0,5 l Wasser versetzt und die flüssige braune Unterphase im Scheidetrichter abgetrennt. Die N-Methylpyrrolidon/Wasser-Oberphase wird mehrmals mit Diethylether extrahiert. Die vereinigten Diethylether-Extrakte und die flüssige braune Unterphase werden zusammen nach Abziehen des Diethylethers im Wasserstrahlpumpenvakuum destilliert. Die Fraktion mit dem Siedepunkt 39 bis 43 °C bei 8 mbar besteht aus farblosem, flüssigem trans-$CF_3CF_2$-CH = CH-$CH_2OH$ und wenig cis-$CF_3CF_2$-CH = CH-$CH_2OH$. Die Ausbeute an trans-$CF_3CF_2$-CH = CH-$CH_2OH$ beträgt 65 Gew.-% der Theorie, an cis-$CF_3CF_2$-CH = CH-$CH_2OH$ 4 Gew.-% der Theorie. Die Verbindungen wurden $^1H$-, $^{13}C$-und $^{19}F$-NMR-spektroskopisch identifiziert.

Beispiel 2

In einem 2-1-Dreihalskolben mit Rückflußkühler, Rührer und Innenthermometer werden 352,8 g (0,7 mol) $CF_3(CF_2)_5$-$CH_2$-CHI-$CH_2OH$, 105 g (1,05 mol) $KHCO_3$ und 1400 ml Dimethylacetamid eingebracht. Die Suspension wird 8 h bei 100 °C gerührt,bis die $CO_2$-Entwicklung beendet ist. Nach Abkühlen auf Raumtemperatur wird vom ausgefallenen KI und überschüssigen $KHCO_3$ abfiltriert. Das Filtrat wird mit etwa 2 l Wasser versetzt und die flüssige braune Unterphase im Scheidetrichter abgetrennt. Die Unterphase wird zehnmal mit je 200 ml Wasser ausgeschüttelt, um das Dimethylacetamid zu entfernen, und danach im Wasserstrahlpumpenvakuum über eine Spaltrohrkolonne destilliert. Die Fraktion mit dem Siedepunkt 85 bis 88 °C bei 9 bis 10 mbar besteht aus farblosem, flüssigem trans-$CF_3(CF_2)_5$-CH = CH-$CH_2OH$ und wenig cis-$CF_3(CF_2)_5$-CH = CH-$CH_2OH$. Die Ausbeute an trans-$CF_3(CF_2)_5$-CH = CH-$CH_2OH$ beträgt 85 Gew.-% der Theorie, an cis-$CF_3(CF_2)_5$-CH = CH-$CH_2OH$ 6 Gew.-% der Theorie. Die Verbindungen wurden $^1H$-, $^{13}C$-und $^{19}F$-NMR-spektroskopisch identifiziert.

Beispiel 3

Beispiel 2 wird wiederholt mit folgenden Abänderungen:
Anstelle von 1400 ml Dimethylacetamid werden 600 ml Dimethylformamid eingesetzt, anstatt 8 h bei 100 °C wird 10 h bei 100 °C gerührt. Die Ausbeute an trans-$CF_3(CF_2)_5$-CH = CH-$CH_2OH$ beträgt 75 Gew.-% der Theorie, an cis-$CF_3(CF_2)_5$-CH = CH-$CH_2OH$ 5 Gew.-% der Theorie.

Beispiel 4

Beispiel 2 wird wiederholt mit folgenden Abänderungen:
Anstelle von 1400 ml Dimethylacetamid werden 700 ml N-Methylpyrrolidon eingesetzt, anstatt 8 h bei 100 °C wird 10 h bei 100 °C gerührt. Die Ausbeute an trans-$CF_3(CF_2)_5$-CH = CH-$CH_2OH$ beträgt 77 Gew.-% der Theorie, an cis-$CF_3(CF_2)_5$-CH = CH-$CH_2OH$ 5 Gew.-% der Theorie. Außerdem liegen wegen der relativ kurzen Reaktionszeit im Destillationsrückstand noch 5 Gew.-% des festen Carbonats der Formel

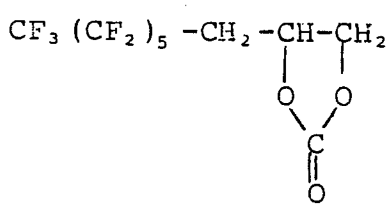

vor, das sich bei längerer Reaktionszeit in die gewünschte Verbindung umwandeln würde.

Beispiel 5

In einem 500-ml-Dreihalskolben mit Rückflußkühler, Rührer und Innenthermometer werden 50,4 g (0,1 mol) $CF_3(CF_2)_5$-$CH_2CHI$-$CH_2OH$, 16,2 g (0,12 mol) $N(CH_3)_4HCO_3$ und 300 ml Acetonitril eingebracht. Die Suspension wird 6 h bei 15 bis 20 °C gerührt und danach 5 h bei 65 °C. Nach Filtration wird das Acetonitril zum großen Teil abgezogen und der flüssige Rückstand mit Wasser versetzt. Die flüssige braune Unterphase wird im Scheidetrichter abgetrennt und im Wasserstrahlpumpenvakuum destilliert. Die Ausbeute an trans-$CF_3(CF_2)_5$-CH = CH-$CH_2OH$ beträgt 78 Gew.-% der Theorie, an cis-$CF_3(CF_2)_5$-CH = CH-$CH_2OH$ 2 Gew.-% der Theorie. Außerdem entsteht etwa 1 Gew.-% der Theorie an Epoxid der Formel

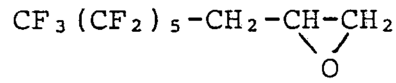

als unerwünschtes Nebenprodukt.

Beispiel 6

In einen 500-ml-Dreihalskolben mit Rückflußkühler, Rührer und Innenthermometer werden 100,8 g (0,2 mol) $CF_3(CF_2)_5$-$CH_2$-CHI-$CH_2OH$, 15,9 g (0,15 mol) $Na_2CO_3$ und 300 ml Dimethylacetamid eingebracht. Die Suspension wird 12 h bei 110 °C gerührt, bis die $CO_2$-Entwicklung beendet ist. Nach Abkühlen auf Raumtemperatur wird der Kolbeninhalt mit ca. 0,5 l Wasser versetzt. Die flüssige braune Unterphase wird ca. zehnmal mit Wasser ausgeschüttelt und im Wasserstrahlpumpenvakuum destilliert. Die Ausbeute an trans-$CF_3(CF_2)_5$-CH = CH-$CH_2OH$ beträgt 77 Gew.-% der Theorie, an cis-$CF_3(CF_2)_5$-CH = CH-$CH_2OH$ 6 Gew.-% der Theorie.

Beispiel 7

Beispiel 6 wird wiederholt mit folgender Abänderung: Anstelle von 15,9 g $Na_2CO_3$ werden 20,7 g (0,15 mol) $K_2CO_3$ eingesetzt. Die Ausbeute an trans-$CF_3(CF_2)_5$-CH = CH-$CH_2OH$ beträgt 82 Gew.-% der Theorie, an cis-$CF_3(CF_2)_5$-CH = CH-$CH_2OH$ 6 Gew.-% der Theorie.

Beispiel 8

In einen 500-ml-Dreihalskolben mit Rückflußkühler, Rührer und Innenthermometer werden 100,8 g (0,2 mol) $CF_3(CF_2)_5$-$CH_2$-CHI-$CH_2OH$, 33,6 g (0,4 mol) $NaHCO_3$ und 300 ml Dimethylacetamid eingebracht. Die Suspension wird 10 h bei 125 °C gerührt, bis die $CO_2$-Entwicklung beendet ist. Die Aufarbeitung erfolgt wie in Beispiel 6. Nach Destillation werden 66 Gew.-% der Theorie an trans-$CF_3(CF_2)_5$-CH = CH-$CH_2OH$ und 6 Gew.-% der Theorie an cis-$CF_3(CF_2)_5$-CH = CH-$CH_2OH$ erhalten.

Beispiel 9

In einen 500-ml-Dreihalskolben mit Rückflußkühler, Rührer und Innenthermometer werden 51,8 g (0,1 mol) $CF_3(CF_2)_5$-$CH_2$-CHI-$CH_2$-$CH_2OH$, 15 g (0,15 mol) $KHCO_3$ und 200 ml Dimethylacetamid eingebracht. Die Suspension wird 8 h bei 95 bis 100 °C gerührt, bis die $CO_2$-Entwicklung beendet ist. Der Kolbeninhalt wird filtriert und das Filtrat mit 0,5 l Wasser versetzt. Die flüssige Unterphase wird im Scheidetrichter abgetrennt, mehrmals mit Wasser ausgerührt und anschließend destilliert. Das farblose flüssige Destillat mit dem Siedepunkt 85 bis 88 °C bei etwa 10 mbar enthält trans-$CF_3(CF_2)_5$-CH = CH-$CH_2$-$CH_2OH$ (Ausbeute: 66 Gew.-% der Theorie), cis-$CF_3(CF_2)_5$-CH = CH-$CH_2CH_2OH$ (Ausbeute: 11 Gew.-% der Theorie) und trans-$CF_3$-$(CF_2)_5$-$CH_2$-CH = CH-$CH_2OH$ (Ausbeute: 6 Gew.-% der Theorie).

Beispiel 10

In einen 1-1-Dreihalskolben mit Rückflußkühler, Rührer und Innenthermometer werden 436,7 g (0,72 mol) $CF_3(CF_2)_7$-$CH_2$-CHI-$CH_2OH$, 108 g (1,08 mol) $KHCO_3$ und 550 ml Dimethylacetamid eingebracht. Die Suspension wird 14 h bei 100 bis 105 °C gerührt,bis die $CO_2$-Ent-wicklung beendet ist. Nach Abkühlen auf Raumtemperatur wird vom KI und unumgesetzten $KHCO_3$ abfiltriert. Das Filtrat wird mit 1 l Wasser versetzt und die flüssige braune Unterphase im Scheidetrichter abgetrennt. Sie wird etwa zehnmal mit Wasser ausgerührt und danach im Wasserstrahlpumpenvakuum über eine Spaltrohrkolonne destilliert. Das Destillat mit dem Siedepunkt 102 bis 110 °C bei 6 bis 8 mbar besteht aus farblosem, flüssigem trans-$CF_3(CF_2)_7$-CH = CH-$CH_2OH$ und wenig cis-$CF_3(CF_2)_7$-CH = CH-$CH_2OH$. Die Ausbeute an trans-$CF_3(CF_2)_7$-CH = CH-$CH_2OH$ beträgt 80 Gew.-% der Theorie, an cis-$CF_3(CF_2)_7$-CH = CH-$CH_2OH$ 6 Gew.-% der Theorie.

In den erfindungsgemäßen Beispielen werden die angestrebten Perfluoralkylalkenole in einer hohen Ausbeute erhalten. Es entstehen, wenn überhaupt, keine nennenswerten Mengen an unerwünschten Nebenprodukten.

Vergleichsbeispiel 1

In einen 500-ml-Dreihalskolben mit Rückflußkühler, Rührer und Innenthermometer werden 100,8 g (0,2 mol) $CF_3(CF_2)_5$-$CH_2$-CHI-$CH_2OH$ und 15,7 g (0,28 mol) KOH in 130 ml Methanol eingebracht. Die Mischung wird 9 h bei Rückflußtemperatur (69 °C) gerührt. Danach wird ca. 2/3 des Methanols bei Normaldruck abdestilliert und der restliche Kolbeninhalt mit Wasser versetzt. Die flüssige Unterphase wird mit Wasser gewaschen und destilliert. Das Destillat mit dem Siedepunkt 79 bis 81 °C bei 7 mbar besteht aus trans-$CF_3(CF_2)_5$-CH = CH-$CH_2OH$ (Ausbeute: 34 Gew.-% der Theorie) und aus $CF_3(CF_2)_5$-$CH_2$-CH(OH)-$CH_2$-$OCH_3$ (Ausbeute: 46 Gew.-% der Theorie).

Vergleichsbeispiel 2

In einen 500-ml-Dreihalskolben mit Rückflußkühler, Rührer und Innenthermometer werden 100,8 g (0,2 mol) $CF_3(CF_2)_5$-$CH_2$-CHI-$CH_2OH$, 14 g (0,25 mol) KOH und 300 ml Dimethylacetamid eingebracht. Der Kolbeninhalt wird 10 h bei 90 bis 100 °C gerührt und danach bei Raumtemperatur mit 0,5 l Wasser versetzt. Die braune Unterphase wird mehrmals mit Wasser ausgerührt und danach destilliert. Die Ausbeute an trans-$CF_3(CF_2)_5$-CH = CH-$CH_2OH$ beträgt 48 Gew.-%, an cis-$CF_3(CF_2)_5$-CH = CH-$CH_2OH$ 3 Gew.-% und an

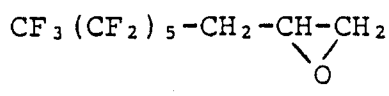

3 Gew.-%. Der relativ große Destillationsrückstand enthält $CF_3(CF_2)_5$-$CH_2$-CH(OH)$CH_2$I, wenig $CF_3(CF_2)_5$-$CH_2$-CH-(OH)-$CH_2OH$ und wenig trans-$CF_3(CF_2)_5$-CH = CH-$CH_2OH$.

In den beiden Vergleichsbeispielen liegt die Ausbeute an dem angestrebten Perfluoralkylpropenol beträchtlich niedriger als in den erfindungsgemäßen Beispielen. In den Vergleichsbeispielen ist zudem eine relativ große Menge an unerwünschten Nebenprodukten, wie teilfluorierten Etheralkoholen (im Vergleichsbeispiel 1) und teilfluorierten Epoxiden (im Vergleichsbeispiel 2) entstanden.

**Ansprüche**

1. Verfahren zur Herstellung von Perfluoralkylalkenolen der nachstehenden Formel I

$R_F$-CH = CH-$(CH_2)_n$-OH ,

worin $R_F$ ein Perfluoralkylrest mit 1 bis 18 C-Atomen und n eine ganze Zahl von 1 bis 6 ist, durch Umsetzung eines Perfluoralkylhalogenhvdrins der nachstehenden Formel II

$R_F-CH_2-CH(Hal)-(CH_2)_n-OH$ ,

worin $R_F$ und n die obengenannte Bedeutung haben und Hal für ein Halogen steht, mit ionischen Basen in Gegenwart eines Lösungsmittels, dadurch gekennzeichnet, daß das Perfluoralkylhalogenhydrin mit einem Alkalimetallcarbonat im Molverhältnis von 1 : 0,5 bis 1,5 oder mit einem Alkalimetallhydrogencarbonat oder einem Alkylammoniumhydrogencarbonat im Molverhältnis von 1 : 1,01 bis 2 in Gegenwart eines aprotischen und polaren Lösungsmittels umgesetzt wird, bis im wesentlichen kein Kohlendioxid mehr entsteht, und aus dem Umsetzungsprodukt die angestrebte Perfluoralkylalkenol-Verbindung isoliert wird, mit der Maßgabe, daß bei Einsatz von Alkalimetallcarbonat und Alkalimetallhydrogencarbonat die Umsetzung bei einer Temperatur von 80 bis 150 °C und bei Einsatz Alkylammoniumhydrogencarbonat die Umsetzung bei einer Temperatur von -5 bis 90 °C durchgeführt wird.

2. Verfahren nach Anspruch 1, dadurch gekennzeichnet, daß das Perfluoralkylhalogenhydrin und das Alkalimetallcarbonat im Molverhältnis von 1 : 0,6 bis 1 eingesetzt werden.

3. Verfahren nach Anspruch 1, dadurch gekennzeichnet, daß das Perfluoralkylhalogenhydrin und das Alkalimetallhydrogencarbonat sowie das Alkylammoniumhydrogencarbonat in einem Molverhältnis von 1 : 1,1 bis 1,5 eingesetzt werden.

4. Verfahren nach einem oder mehreren der Ansprüche 1 bis 3, dadurch gekennzeichnet, daß bei Einsatz von Alkalimetallcarbonat und Alkalimetallhydrogencarbonat die Umsetzung bei einer Temperatur von 90 bis 130 °C durchgeführt wird.

5. Verfahren nach einem oder mehreren der Ansprüche 1 bis 4, dadurch gekennzeichnet, daß bei Einsatz von Alkylammoniumhydrogencarbonat die Umsetzung bei einer Temperatur von -5 bis 90 °C durchgeführt wird, wobei im Falle eines Perfluoralkylhalogenhydrins mit n = 2 bis 6 eine Umsetzungstemperatur von 40 bis 90 °C eingehalten wird und im Falle von n = 1 zunächst eine Umsetzungstemperatur von -5 bis 25 °C eingehalten wird, bis das Perfluoralkylhalogenhydrin im wesentlichen verbraucht ist und anschließend bei einer Temperatur von 40 bis 90 °C gehalten wird, bis im wesentlichen kein Kohlendioxid mehr entsteht.

6. Verfahren nach einem oder mehreren der Ansprüche 1 bis 5, dadurch gekennzeichnet, daß $R_F$ ein Perfluoralkylrest mit 2 bis 10 C-Atomen ist.

7. Verfahren nach einem oder mehreren der Ansprüche 1 bis 6, dadurch gekennzeichnet, daß n = 1 ist.

8. Verfahren nach einem oder mehreren der Ansprüche 1 bis 7, dadurch gekennzeichnet, daß als Lösungsmittel Dimethylformamid, Dimethylacetamid, N-Methylpyrrolidon oder Acetonitril eingesetzt wird.

9. Verfahren nach einem oder mehreren der Ansprüche 1 bis 7, dadurch gekennzeichnet, daß bei Einsatz von Alkalimetallcarbonat und Alkalimetallhydrogencarbonat Dimethylformamid, Dimethylacetamid, oder N-Methylpyrrolidon und bei Einsatz von Alkylammoniumhydrogencarbonat Acetonitril als Lösungsmittel eingesetzt wird.